# EUROPEAN PATENT APPLICATION

(11) **EP 4 193 974 A1**
(43) Date of publication of application: **14.06.2023**
(21) Application number: 22020219.6
(22) Date of filing: 10.05.2022
(51) Int. Cl.: A61F 9/008

(54) **SCANNING OPHTHALMIC TRANSSCLERAL LASER PROBE SYSTEM**

(30) Priority: 13.12.2021 US 202163265319 P; 09.05.2022 US 202217739973
(71) Applicant: Mehmet Melek, Istanbul 34457 (TR)
(72) Inventor: Mehmet Melek, Istanbul 34457 (TR)

(57) **Abstract**

A multiple-fiber scanning ophthalmic transscleral laser probe system capable of firing multiple laser spots sequentially on the perilimbal area through the use of multiple fibers and an optical switching mechanism is disclosed. The design aims to reduce probe motion on the surface of the eye during transscleral cyclophotocoagulation and pulsed transscleral laser therapy by allowing multiple laser shots to be fired sequentially in a partial circular pattern without any probe movement and without the use of moving parts inside the probe. Sequential firing from a fixed probe location allows precise power level for each treatment spot and prevents the probe tip getting caught on or damaging the conjunctiva.

## Description

### FIELD OF THE INVENTION

The present invention consists of an ophthalmic laser probe system which aims to simplify transscleral cyclophotocoagulation and pulsed transscleral laser therapy for glaucoma by the use of a scanning probe placed on the limbus or perilimbal area with the use of an optical switching mechanism and multiple fibers. The system replaces the sweeping or repositioning motion of existing probes by sequential shots through multiple fibers, allowing the probe to be held in a fixed position to cover at least the treatment of one quadrant of the eye.

### BACKGROUND

Fiber optic laser probes are commonly used to transmit laser energy for medical applications. Transscleral cyclophotocoagulation and pulsed transscleral laser therapy involve the placement of a fiber optic probe over the surface of the eye on the limbus or perilimbal area, with the fiber tip approximately 0-3 mm away from the limbus in order to transsclerally emit laser radiation towards the ciliary processes.

"Transscleral cyclophotocoagulation" aims to destroy some of the ciliary processes in order to reduce intraocular pressure by applying continuous wave laser energy to a series of selected points (Gaasterland D.,et al, "Initial Experience with a New Method of Laser Transscleral Cyclophotocoagulation for Ciliary Ablation in Severe Glaucoma", Trans Am Ophthalmol Soc 90:225-246,1992) whereas "pulsed transscleral laser therapy" uses a pulsed laser with short on/off time cycles, with the probe moved in a sweeping motions in the upper and lower perilimbal semi-circles (Williams A.L., et al, "Clinical Efficacy and Safety Profile of Micropulse Transscleral Cyclophotocoagulation in Refractory Glaucoma", J Glaucoma 2018;27: 445-449).

Existing probes are either moved in steps or in a sweeping motion to cover the necessary portion of the treatment area. The proximal end of the probe is attached to an ophthalmic photocoagulation laser which generates a laser beam while the distal end is placed on the perilimbal area. Laser shots have either a destructive (continuous wave) or non-destructive (pulsed, tissue-sparing) effect on the ciliary processes transsclerally for the treatment of glaucoma.

Existing probe designs have a shaped tip containing a single laser fiber (e.g. U.S. Patent 5,372,595 to Gaasterland, et al, U.S. Patent 8,945,103 to Chew, et al, U.S. Patent 9,700,461 to Buzawa, et al), or a single laser fiber with an additional illumination fiber (e.g. U.S. Patent 9,629,749 to Vold, et al) as well as more basic rounded fiber tips without surrounding footplate. The concept of a replaceable tip has also been considered (e.g. U.S. Patent 10,758,118 to Chen, et al). While continuous wave transscleral laser cyclophotocoagulation which destroys some of the ciliary processes has been practiced for decades, tissue-sparing transscleral laser therapy with short duration laser pulses has become common practice in recent years. The outcome of both treatment modalities is dependent on the surgeon's manual skills, with the probe tip getting caught on or damaging the conjunctiva during repositioning or sweeping motion presenting an occasional hurdle.

### SUMMARY

- The primary object of the invention is to reduce probe motion on the surface of the eye by providing a fixed probe setup for at least a quadrant of the treatment area.
- A second object is the placement of spots on the perilimbal area in an accurate predictable pattern for improved medical outcome.
- A third objective is to prevent the probe tip getting caught on or damaging the conjunctiva during a sweep motion.
- A fourth object is the provision of such an apparatus which is readily usable with existing laser equipment
- A fifth object is the provision to use such an apparatus attached to existing laser systems for the purpose of firing a series of spots in rapid sequence to form a treatment pattern on the perilimbal area.

### BRIEF DESCRIPTION OF THE DRAWINGS

FIG. 1 is a schematic description of the preferred embodiment of the invention
FIG. 2A shows a probe with a flat treatment surface for the treatment of a quadrant
FIG. 2B shows a probe with protruding round-ended fiber optic tips for the treatment of a quadrant
FIG.3A shows a probe with a flat treatment surface for the treatment of a semi-circle
FIG. 3B shows a probe with protruding round-ended fiber optic tips for the treatment of a semi-circle
FIG. 4A shows the positioning of the probe on the eye for treating a quadrant
FIG. 4B shows the positioning of the probe on the eye for treating a semi-circle
FIG. 4C shows the positioning of the probe on the eye for treating two semi-circles, leaving the nasal and temporal sections untreated
FIG. 4D shows the positioning of the probe on the eye with the capability of treating 360 degrees, while leaving the selection of the treatment area to the optical switching system

### DETAILED DESCRIPTION

An ophthalmic transscleral laser probe is an instrument whose proximal end attaches to an ophthalmic photocoagulating laser via a connector and its distal end is placed in contact with the perilimbal area of the eyeball for the purpose of transferring laser energy to treat ophthalmic tissue. The most common applications of the transscleral laser are transscleral cyclophotocoagulation and pulsed transscleral laser therapy, i.e. the destructive or non-destructive heating of the ciliary processes with laser light. Transscleral cyclophotocoagulation typically shoots 10-22 laser shots with the probe placed at distinct intervals, whereas pulsed transscleral laser therapy use a sweeping motion over the lower and upper perilimbal areas, leaving the nasal and temporal sections untreated.

Prior art transscleral probes have a single laser firing fiber (and sometimes an illumination fiber) but lack the scanning ability unique to the invention. Prior art transscleral laser probes have a single connector 14, a single optical fiber 12, a protective tubing 10 to mechanically protect the fiber, a handpiece 16 for the surgeon to hold the probe tip pressed onto the eyeball in the perilimbal region. Some models have multiple fibers 12, with one fiber to carry the laser beam and the others to carry an illumination component.

An optical switching mechanism 22 (prior art) is a commercially available device with one input socket 30 and several output sockets 28. Laser energy entering the switch via the input socket 30 is diverted to one of the output sockets 28.

Electronic control of the device allows switching from one output socket to another.

The apparatus in the present invention has a similar structure as prior art commercially available transscleral laser probes, but contains multiple connectors 14, multiple laser fibers 12 and a multiple fiber holding piece 18 at the tip, with all fibers 12 attached to an optical switching mechanism 22 through several connectors 14. The optical switching mechanism 22 is itself connected to the output 20 of the laser unit 26 via a fiber optic cable 24. Laser power from the laser unit 26 is sequentially distributed to the output sockets 28 and to the probe through the connectors 14 and the optical fibers 12. The optical switching mechanism 22 allows electronic control of parameters such as the selection of output socket 28 and the duration of the laser shot at each socket.

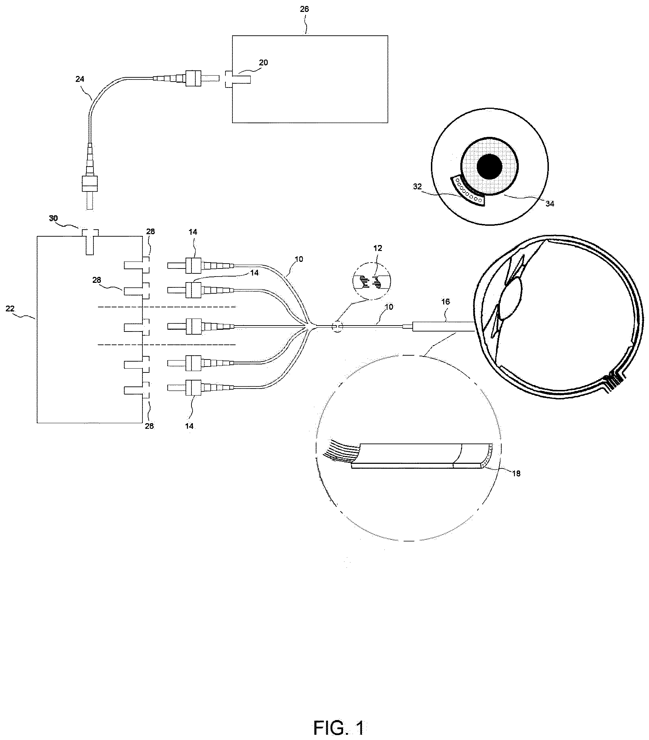

The novelty of the invention is its capability to shoot several laser spots sequentially on the perilimbal area 34 in a pattern 32 as shown in FIG.1 through the tip 18. Treating a quadrant would typically require 8-16 spots and hence optical fibers, though other quantities are also feasible. Such an automated sequence of spots offers the possibility to have consistent laser delivery independent of the surgeon's hand. Compared to simultaneous multiple shots through a power divider, the sequential approach allows accurate control of the laser power in each individual optical fiber 12 and the use of lower total output power from the laser unit 26.

The surgeon will shoot a treatment pattern 32, followed by a relocation of the probe tip 18 to the next quadrant or semi-circle treatment location. This will be followed by another treatment sequence, with the process repeated until completion of the treatment for the whole eye.

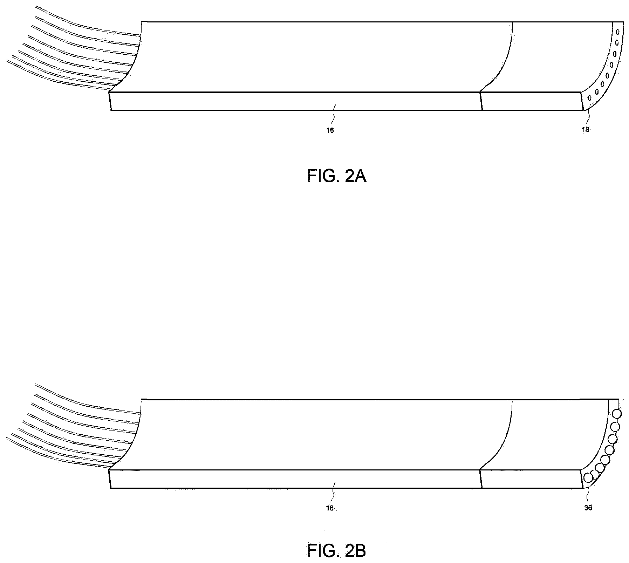

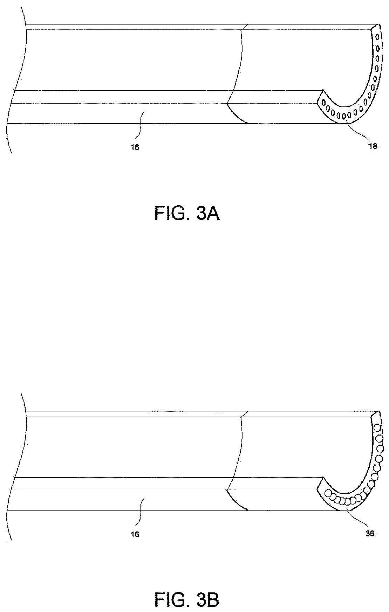

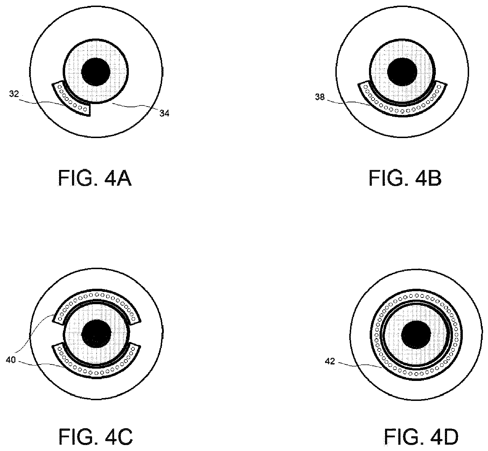

The preferred embodiment of the invention is represented in FIG.1. The apparatus includes several connectors 14 for attachment to an ophthalmic photocoagulation laser 26 via an optical switching mechanism 22, several individual fibers 12 each attached to one connector 14, protective tubings 10 to protect the fibers from external damage, a handpiece 16 to be held by the surgeon and a tip 18 (FIG. 2A) containing multiple fibers 12, polished flush to the tip surface. The use of this embodiment will result in a sequential treatment pattern 32 on the perilimbal area 34 covering a quadrant (FIG. 4A), with as many spots as individual fibers 12 and all laser shots being fired in sequence.
A second embodiment with identical features except for a tip 36 with protruding fibers, with rounded, spherical, flat or otherwise shaped ends is shown in FIG.2B. Such an embodiment will allow multiple spots to be fired sequentially with the probe tips pressing over the perilimbal area 34 which may result in more efficient treatments compared to the non-protruding probe tip 18.
A third embodiment has a semi-circular tip (FIG. 3A, FIG. 3B), allowing the treatment of a semi-circular area 38 (FIG. 4B)
A fourth embodiment has two semi-circular tips allowing to treat the upper and lower semi-circles 40, leaving out the nasal and temporal sections (FIG. 4C)
A fifth embodiment has a 360 degree tip covering the whole perilimbal area 42, with the selection of the treatment area defined by the optical switching system (FIG. 4D)

### REFERENCES

### PUBLICATIONS

Gaasterland D, Pollack I., "Initial Experience with a New Method of Laser Transscleral Cyclophotocoagulation for Ciliary Ablation in Severe Glaucoma", Trans Am Ophthalmol Soc 90:225-246,1992

Williams AL, Moster MR, Rahmatnejad K, Resende AF, Horan T, Reynolds M, Yung E, Abramowitz B, Kuchar S, Waisbourd M, "Clinical Efficacy and Safety Profile of Micropulse Transscleral Cyclophotocoagula-tion in Refractory Glaucoma", J Glaucoma 2018;27: 445-449

### PATENTS

U.S. Patent 5,372,595 Gaasterland, et al, "Contact probe for laser cyclophotocoagulation", December 13, 1994

U.S. Patent 8,945,103 Chew, et al, "Contact probe for the delivery of laser energy", February 3, 2015

U.S. Patent 9,700,461 Buzawa, et al, "Convex contact probe for the delivery of laser energy", July 11, 2017

U.S. Patent 9,629,749 Vold, et al, "Illuminated treatment probe for delivering laser energy", April 25, 2017

U.S. Patent 10,758,118 Chen, et al, "Handheld ophthalmic laser system with replaceable contact tips and treatment guide", September 1, 2020

## Claims

1. A scanning ophthalmic transscleral laser probe system capable of shooting several laser spots on the perilimbal area sequentially in a partial circular pattern comprising :
- an optical switching mechanism connected to the ophthalmic photocoagulation laser
- a transscleral laser probe structure with multiple fibers, each with its own connector
- tubings to protect and hold the fibers
- a handpiece for the surgeon to hold
- a tip for pressing on the eye surface perilimbal area, containing the multiple fibers to treat a quadrant

2. The system of claim 1 with a probe tip with protruding fibers, with rounded, spherical, flat or otherwise shaped tips

3. The system of claim 1, with a tip for pressing on the eye surface perilimbal area, containing the multiple fibers to treat a semi-circle

4. The system of claim 2, with a tip for pressing on the eye surface perilimbal area, containing the multiple fibers to treat a semi-circle

5. The system of claim 1, with a tip for pressing on the eye surface perilimbal area, containing the multiple fibers to treat and upper and lower semi-circles, leaving out the nasal and temporal sections

6. The system of claim 2, with a tip for pressing on the eye surface perilimbal area, containing the multiple fibers to treat and upper and lower semi-circles, leaving out the nasal and temporal sections

7. The system of claim 1, with a tip for pressing on the complete perilimbal eye surface, containing the multiple fibers over a 360 degree range, with the selection of the treatment area defined by the optical switching system

8. The system of claim 2, with a tip for pressing on the complete perilimbal eye surface, containing the multiple fibers over a 360 degree range, with the selection of the treatment area defined by the optical switching system
